**Europäisches Patentamt**

**European Patent Office**

**Office européen des brevets**

(11) Publication number: **0 112 940**
**B1**

⑱

(12) **EUROPEAN PATENT SPECIFICATION**

(45) Date of publication of patent specification: **15.10.86**

(21) Application number: **82306984.4**

(22) Date of filing: **30.12.82**

(51) Int. Cl.⁴: **A 61 K 37/465, C 12 N 9/00**

(54) Method of producing a tissue plasminogen activator and composition comprising same.

(43) Date of publication of application:
**11.07.84 Bulletin 84/28**

(45) Publication of the grant of the patent:
**15.10.86 Bulletin 86/42**

(84) Designated Contracting States:
**BE CH DE FR GB IT LI NL SE**

(56) References cited:
**FR-A-1 347 029**
**US-A-3 555 000**
**US-A-3 904 480**
**US-A-3 998 947**
**US-A-4 245 051**

(73) Proprietor: **KOWA COMPANY, LTD.**
**6-29, 3-chome Nishiki**
**Naka-ku Nagoya-shi Aichi-ken (JP)**

(72) Inventor: **Yoshizaki, Hideo**
**1-4-9, Sayamadai**
**Sayama-shi Saitama-ken (JP)**
Inventor: **Mori, Toshihito**
**4-16-3, Fujimi-cho**
**Higashi-Murayama-shi Tokyo (JP)**

�titleRepresentative: **Webb, Frederick Ronald et al**
**G.F. Redfern & Co. Marlborough Lodge 14**
**Farncombe Road**
**Worthing West Sussex BN11 2BT (GB)**

Courier Press, Leamington Spa, England.

EP 0 112 940 B1

**Description**

This invention relates to a method of producing a tissue plasminogen activator (hereinafter referred to simply as "TPA") and to a composition comprising TPA. More particularly, the invention relates to a method of producing TPA conveniently by which TPA is stabilized by addition of albumin and also to a composition in which TPA maintains its activity stably, even when preserved over an extended period of time.

It is well known that urokinase, which is extracted and purified from human urine or a tissue culture medium of kidney cells, is useful as a plasminogen activator. However, urokinase has the drawback that despite its capability of dissolving thrombus, urokinase is lowered in its activity by fibrinogen, $\alpha_2$-plasmin and plasminogen in blood, thereby inducing bleeding or tachyphylaxis when administered into blood.

In recent years, it has been found that TPA which is free of the drawback of urokinase is existent, as a plasminogen activator, in the internal organs, vessel walls, body fluid of human beings or animals, tissue culture media of these cells or cancer cells, or culture media of microorganisms having TPA-producing efficacy by genetic engineering. There is therefore a continuing need for the development of TPA as a drug.

TPA is different from urokinase in its immunological activity and affinity for fibrin. TPA does not react with an antibody for urokinase but combines firmly with fibrin with the advantage that it develops strong activity in blood as an activator in the presence of fibrin. Accordingly, TPA is greatly expected to be a substitute for urokinase which exhibits suitable thrombus-dissolving activity even in smaller amounts and which does not accompany the side-effects experienced with urokinase.

TPA has heretofore been produced by isolation from the internal organs, vessel walls, body fluid of human beings or animals, tissue culture media of these cells or cancer cells, or culture media of microorganisms having TPA-producing efficacy by genetic engineering. Known techniques for the isolation and purification of TPA include salting-out, ion-exchange, chromatography, gel filtration, hydrophobic chromatography and affinity chromatography. Among these techniques, affinity chromatography is the most excellent in which use is made, as the ligand, of fibrin [Biochimica Biophysica Acta, *621*, 241 (1980)], arginine [Thrombos Haemostas, *42*, 414 (1979)] and lysine [Arch. Biochem. and Biophys., *189*, 185 (1978)].

However, TPA is prone to decrease in its activity as it is purified. Highly purified TPA can only exert extremely reduced activity. Research has been conducted for stabilizers which might prevent TPA against its activity reduction, and some articles report that arginine [J.B.C. *254*, 1998 (1979)], gelatin [Biochemistry, *8*, 79 (1969)] and fibrin [Thrombos Haemostas *45*, 43 (1981)] are effective stabilizers for the protein.

Arginine and gelatin are still unsatisfactory in their efficacy, whereas fibrin exhibits an excellent stabilizing effect but is not pharmaceutically acceptable. Therefore, these stabilizers are far from practically useful.

Under these circumstances, the present inventors have made many studies of stabilizing TPA. Though unstable in higher purity, TPA shows a substantial level of stability in a crude enzyme solution of low purity. In the studies leading to the present invention, it has been discovered that serum albumin is effective to stabilize TPA in a TPA-containing crude enzyme solution separated from a perfused swine ear. The stabilizing effect of albumin on TPA has been almost equal to that of fibrin and substantially higher by over 100 times than those of gelatin and protamine sulfate which are known as excellent stabilizers for urokinase.

The present invention accordingly provides a method of producing TPA in which albumin is added at an arbitrary stage of purifying or drying TPA, whereby the difficulties of the prior art techniques are overcome.

The invention also provides a composition comprising TPA and albumin in which the activity of TPA is stably retained.

Albumin useful as a stabilizer for the practice of the present invention may be of any origins, among which albumin derived from human serum or placental is preferably utilized.

The method of the invention can be suitably applied to any processes for isolating and purifying TPA from crude enzyme solutions which are prepared from various starting materials. Such isolation and purification processes include dialysis and ultrafiltration which may be used singly or in combination, coupled with affinity chromatography which is particularly preferable. Albumin may be added directly to the crude enzyme solutions or at any one selected stage of the production of TPA.

In the case where TPA originates from tissue culture media of normal and cancer cells and organ extracts such as of swine, these media and extracts should be heated at 60°C for about 10 hours for killing viruses. However, the heating treatment causes TPA to be deactivated. It is therefore preferable to add albumin prior to the heat treatment with the result that heating hardly affects the activity of TPA.

The amount of albumin to be added at the purification stage varies depending on the type of treatment and is generally in the range of 0.001 to 10% (w/v) based on the crude enzyme solution.

TPA thus isolated and purified is freeze-dried or spray-dried to obtain a powdery product. This drying treatment also tends to deactivate TPA and hence needs the addition of albumin. In such instance, albumin is added while taking account of the content in the final product and is generally in the range of 0.003 to 5% (w/v). If albumin which has been added at the purification stage is present in excess in the final product, it is removed by any conventional technique to a suitable extent of concentration and then dried to obtain a powdery product.

When preserved over a long period of time, TPA involves reduced activity. However, the addition of albumin suppresses the activity reduction. The amount of albumin to be added is in the range of 0.003 to 1% (w/v). Albumin may be added at a step of purifying, drying or powdering TPA, or freshly to the final TPA product.

The composition according to the invention which comprises TPA produced above and albumin is not only much more stable than those compositions containing other types of stabilizers, but also requires albumin to be added in very small amounts. The composition of the invention is thus excellent as a drug.

The above disclosure generally describes the present invention. A more complete understanding can be obtained by reference to the following specific examples which are provided herein for purposes of illustration only and are not intended to be limiting.

## Example 1

Stability Test of TPA

(1) Stability in Solutions

To aliquots of a physiological saline aqueous solution of TPA (60 U/ml) were added the stabilizers indicated in Table 1. The mixtures were allowed to stand at room tempeature for 1 to 2 days and provided for measurement of the residual activities of TPA.

Titration was effected by the following method both in this example and in the ensuing examples.

An agar-added fibrin plate was made using, as a starting material, 75% clottable fibrinogen (made by Miles Inc. plasminogen content: about 140 casein units/g of coagulated protein). The fibrinolytic activity of TPA was determined in terms of its response in comparison to that of urokinase. A tissue activator solution was diluted with a 0.067 M tris-hydrochloric acid buffer solution (pH 8.0) containing 1% gelatin, 0.1 M sodium chloride and 0.1% sodium azide and applied as a drop of 5 μl to the plate. The activity of TPA was calculated from the dilution factor required to obtain the same lysis zone as in the case with 5 μl of a urokinase standard solution of 1.5 IU/ml in the same buffer as used for TPA.

The results are shown in Table 1.

0 112 940

TABLE 1

| Stabilizer | Final Conc. (%) | Residual Activity (%) | |
| | | After 1 day | After 2 day |
|---|---|---|---|
| Human serum albumin | 0.001 | 33 | 0 |
| | 0.003 | 100 | 100 |
| | 0.01 | 100 | 100 |
| | 0.1 | 100 | 100 |
| Fibrin | 0.001 | 75 | 33 |
| | 0.003 | 100 | 100 |
| | 0.01 | 100 | 100 |
| | 0.1 | 100 | 100 |
| Gelatin | 0.1 | 17 | 0 |
| | 0.3 | 67 | 42 |
| | 0.5 | 100 | 100 |
| Fibrinogen | 0.1 | 100 | 100 |
| Protamine sulfate | 0.5 | 75 | 33 |
| Dextran T—40 | 1 | 25 | 0 |
| Mannitol | 3 | 25 | 0 |
| Control | — | 16 | 0 |

(2) Stability on Freeze-drying

To aliquots of a physiological saline aqueous solution of TPA (60 U/ml) were added the stabilizers indicated in Table 2, and the mixtures were adjusted in their pH to 7.0. The samples were preliminarily frozen at a temperature ranging from −40 to −50°C for 3 hours and then subjected to primary drying at a temperature ranging from −40 to +30°C under a vacuum of 5,32 × 10$^{-4}$ to 7,98 × 10$^{-4}$ mbar (0.4 × 10$^{-3}$ to 0.6 × 10$^{-3}$ mmHg) for 2 hours and subsequently to secondary drying at 30°C under a vacuum of 1,33 × 10$^{-4}$ to 2,66 × 10$^{-4}$ mbar (0.1 × 10$^{-3}$ to 0.2 × 10$^{-3}$ mmHg) for 3 hours. The residual activities of the resulting powder were compared to determine the stabilizing effect of each of the test stabilizers.

4

The results are shown in Table 2.

TABLE 2

| Stabilizer | Final Conc. (%) | Residual Activity (%) |
|---|---|---|
| Human serum albumin | 0.001 | 72 |
| | 0.1 | 95 |
| | 0.5 | 100 |
| Gelatin | 0.01 | 65 |
| | 0.1 | 82 |
| | 0.5 | 85 |
| Dextran | 1 | 96 |
| Protamin sulfate | 0.5 | 58 |
| Mannitol | 3 | 83 |
| Control | — | 71 |

(3) Stability on Virus-killing Treatment

To aliquots of a physiological saline aqueous solution of TPA (60 U/ml) wre added the stabilizers indicated in Table 3, and the mixtures were adjusted in their pH to 7.0. The samples were thermally treated at 60°C for 10 hours and measured in terms of their residual activities for comparatively determining the stabilizing effect of each of the test stabilizers.

The results are shown in Table 3.

TABLE 3

| Stabilizer | Conc. (%) | Residual Activity (%) |
|---|---|---|
| Human serum albumin | 0.001 | 30 |
| | 0.003 | 34 |
| | 0.01 | 62 |
| | 0.1 | 100 |
| | 0.5 | 100 |
| Gelatin | 0.1 | 28 |
| | 0.5 | 50 |
| Control | — | 23 |

## Example 2

A pig's ear was perfused with Tyrode's solution added to it intermittently with acetylcholine (about 1 μg) to allow an activator of its vascular wall to be released. The perfusin liquid was fractionated in a fraction collector to collect a fraction of higher fibrinolytic activity. As a result, there was obtained about 500 ml of a solution per pig's ear.

100 pig's ears (400 g/ear on the average) were perfused to obtain a solution of about 50 l (3U/ml). The solution was mixed with 300 g/l of ammonium sulfate, adjusted in its pH to 7.0 and allowed to stand overnight. The resulting precipitate was separated by filtration using Celite® and charged on a column (4 × 25 cm) along with the Celite®. The column was washed with a 0.01 M phosphate buffer solution (pH 7.2) containing 2 M ammonium sulfate and 1 M sodium chloride, whereupon the activator was eluted at a concentration gradient of from the buffer solution just stated through a 0.01 M phosphate buffer solution (pH 7.2) containing 1 M sodium chloride. The resulting eluate was found to have a volume of 1 l, an activity of 50 U/ml and a specific activity of 125 U/A280. The solution was subjected to adsorption of an octyl-Sepharose® column (2.5 × 10 cm), after which ethylene glycol was increased up to about 50% by a concentration gradient technique to elute an activator. The eluate has a volume of 3 l an activity of 25 U/ml and a specific activity of 900 U/A280.

One liter of the thus obtained solution was used and admixed with human serum albumin at a rate of 100 g/ml (0.01%), followed by adsorption on a fibrin-Sepharose® column (2 × 10 cm), sufficient washing with a 1 M sodium chloride solution and elution with a 0.005 M phosphate buffer solution (pH 7.2) containing 0.01% human serum albumin and 0.5 M arginine. The resulting solution had a volume of 230 ml and an activity of 110 IU/ml. The solution was concentrated by means of Diafilter® A—15T and subjected to gel filtration on a Sephadex® G—150 column (3 × 100 cm) which has been equilibrated with a 0.01 M phosphate buffer solution (pH 7.0) containing 0.01% human serum albumin, 1.5 M sodium chloride and 0.01 M EDTA, thereby collecting an active fraction containing the activator. The fraction was dialyzed against a physiological saline solution. The resulting solution had a volume of 70 ml and an activity of 300 IU/ml. The specific acitivity of the solution from which albumin added had been removed was found to be 17,000 U/A280 (yield 42%).

In the same manner, 1 l of the solution was used and treated without use of albumin. The solution had a volume of 130 ml, an activity of 80 U/ml and a specific activity of 12,000 U/A280 (yield 28%).

## Example 3

Five kilograms of pig's hearts were ground in a meat grinder and acetone was added (−29°C) at a rate of 2 l/kg, followed by agitation in a blender and filtration. This defatting procedure was repeated several times to obtain 700 g of an acetone-treated powder. The powder was mixed with 800 ml of a 0.3 M potassium acetate solution (pH 4.2) per 100 g of powder, followed by agitation in a blender, thereby extracting a tissue activator at 4°C for 3 hours. The resulting extract was collected by centrifugal separation, and the residue was re-extracted by addition of a 0.3 M potassium acetate solution (pH 4.2) in an amount of 400 ml/100 g. The re-extract was admixed with ammonium sulfate at a rate of 300 g/l, adjusted in its pH to 7.0 and allowed to stand overnight at 4°C. The resulting precipitate was collected by centrifugal separation, dissolved in a 0.3 M potassium acetate solution (pH 4.2) and adjusted in its pH to 7.0 to obtain a crude enzyme solution. The solution had a volume of 2 l and an activity of 300 U/ml. To the solution was added albumin according to the procedure of Example 2, purified by affinity chromatography on a fibrin-sepharose® column and dialyzed using a 0.001 N hydrochloric acid solution containing 0.9% sodium chloride. Thereafter, neutralization was effected with use of an aqueous sodium hydroxide solution to obtain a solution having a volume of 1 l, an activity of 350 U/ml and a specific activity of 8,000 U/A280.

## Example 4

Blood was collected, using a sodium citrate solution as an anticoagulant, from health volunteers just compressed vascularly by means of a hemadynamometer. Immediately after being collected, the blood was centrifuged at 3,000 rpm for 10 minutes to give 1 l of blood plasma in total. The plasma was mixed with benzamidine and EDTA each in an amount of 5 mM, followed by adsorption on a lyzine-Sepharose® column (3 × 25 cm) over each of three divided portions. Each column was sufficiently washed with a 0.005 M phosphate buffer solution (pH 7.4) containing 0.6 M sodium chloride and eluted with a 0.005 M-phosphate buffer solution (pH 7.4) containing 1.5 M sodium chloride. The resulting eluate was diluted to a factor of three times with water and admixed with albumin according to the procedure of Example 2, followed by purification by affinity chromatography on a fibrin-Sepharose® column and concentration by ultrafiltration. The resulting solution had a volume of 4 ml, an activity of 30 U/ml and a specific activity of 3,000 U/A280.

## Example 5

Cells isolated from human tumor cells were sufficiently grown in a DME medium to which 100 U/ml of penicillin G and 100 γ/ml of streptomycin were added and then in a medium admixed with 20% of FCS. Thereafter, the culture solution was cultivated in a FCS-free medium at 37°C for 30 hours. At this stage, the resulting solution was found to have an activity of about 10 U/ml. One liter of the culture solution was separated by centrifuge at 5,000 rpm for 20 minutes. The resulting supernatant liquid was treated in the

# 0 112 940

same manner as in Example 2 to obtain a final tissue activator having a volume of 80 ml and an activity of 50 U/ml.

## Claims

1. A method of producing a tissue plasminogen activator, which comprises adding albumin at a stage of purifying or drying said activator.

2. A composition comprising a tissue plasminogen activator and albumin in such a manner that said activator retains its activity stably.

## Patentansprüche

1. Verfahren zur Herstellung eines gewebeplasminogen Aktivators wobei ein Zusetzen von Albumin waehrend der Reinigung oder Trocknung des Aktivators stattfindet.

2. Eine Zubereitung die einen gewebeplasminogen Aktivator und Albumin derartig enthaelt, dass der Aktivator seine Aktivitaet in stabiler Weise beibehaelt.

## Revendication

1. Procédé de production d'un activateur plasminogène de tissu, comprenant l'addition d'albumine dans une étape de purification ou de séchage de l'activateur.

2. Composition comprenant un activateur plasminogène de tissu et de l'albumine, de façon que l'activateur conserve une activité stable.